# EUROPEAN PATENT SPECIFICATION

(11) **EP 2 742 452 B1**
(45) Date of publication and mention of the grant of the patent: **06.11.2019**
(21) Application number: 12772520.8
(22) Date of filing: 14.09.2012
(51) Int. Cl.: G16H 20/30, G16H 20/60, G16H 50/20

(54) **LIFE RHYTHM PROCESSING SYSTEM FOR RECOMMENDING ACTIVITIES**
LEBENSRHYTHMUSVERARBEITUNGSSYSTEM ZUM VORSCHLAGEN VON AKTIVITÄTEN
SYSTÈME D'ANALYSE DU RYTHME DE VIE POUR RECOMMANDER DES ACTIVITÉS

(30) Priority: 27.09.2011 JP 2011211229
(43) Date of publication of application: 18.06.2014
(73) Proprietor: Sony Corporation, Tokyo 108-0075 (JP)
(72) Inventor: SAKAMOTO, Takayuki, Tokyo 108-0075 (JP); TAKAGI, Tsuyoshi, Tokyo 108-0075 (JP); IKENOUE, Shoichi, Tokyo 108-0075 (JP)
(74) Representative: D Young & Co LLP
(86) International application number: PCT/JP2012/005891
(87) International publication number: WO 2013/046589

(56) References cited:
- WO-A1-00/52604
- WO-A2-2006/079942
- WO-A2-2008/001366
- GB-A- 2 454 705
- US-A- 5 673 691
- US-A1- 2006 183 980
- US-A1- 2008 086 318
- US-B2- 7 261 690

## Description

### TECHNICAL FIELD

This technique relates to terminal devices, external devices, information processing methods, programs, and information processing systems. More particularly, this technique facilitates use of information that matches the life rhythm of a user at a desired time.

### BACKGROUND ART

There have been health services that provide each user with health-related information by using an information processing system. For example, a terminal device having a communication function is linked to a measuring apparatus that measures vital data (such as weights, blood pressures, numbers of steps), and clients' vital data measured by the measuring apparatus is recorded on an external device by the terminal device. The external device performs statistical processing or the like on the recorded vital data, and transmits the results of the statistical processing to the terminal device.

As another health service, the health management system suggested in Patent Document 1 provides advice on food, exercise, and the like, based on lifestyle data and vital data.

### CITATION LIST

### PATENT DOCUMENT

Patent Document 1: Japanese Patent Application Laid-Open No. 11-47096
Other previously proposed arrangements are disclosed in WO 2008/001366 and US 5673691.

### SUMMARY

### PROBLEMS TO BE SOLVED

Through the service that reports the results of statistical processing of vital data, changes in the stored vital data can be grasped. If daily changes in the vital data are small, however, changes in the reported results of the statistical processing are also small. As a result, the interest level of the users might become lower, and the use of the service might become less frequent.

In a case where a search system that can provide information desired by a user is used, the user cannot obtain information unless he/she sets various kinds of search criteria. Therefore, once the user determines that the operation is complicated, it is difficult for the user to increase the frequency of use of the service or to regularly use the service.

Further, through a service that automatically reports only the information corresponding to the present time like a television or radio broadcasting system, a user cannot reuse missed information or plan a schedule or the like by obtaining information corresponding to a future time. Therefore, the usefulness of the service becomes lower.

In view of this, this technique aims to facilitate use of information that matches the life rhythm of a user at a desired time.

### Solution to Problem

The present invention discloses an information processing system, information processing method and computer program product as defined by claims 1-14. According to a first example, the disclosure is directed to an information processing system that includes a user interface that receives an input by a user; a life rhythm analyzing unit that analyzes the user's life rhythm based on information collected by a sensor for the analysis; and a content transmitting unit that acquires the input and analysis result of the life rhythm of the user from the life rhythm analyzing unit and provides content based on the input and the analysis result.

The input may correspond to any one of information indicating a physical attribute of the user, information indicating content of interest to the user, and a selection of at least one of a plurality of categories of content, and the content transmitting unit acquires the content to be provided based on the input and the analysis result.

The input may correspond to at least one of a date and time, and the content transmitting unit acquires the content to be provided based on the at least one of the date and time and the analysis result. The content transmitting unit may determine an action probability based on the at least one of the date and time and the analysis result, and the determined action probability may correspond to at least one of exercising, eating and sleeping. The content transmitting unit may determine a category of content based on the determined action probability, and the content transmitting unit may acquire the content to be provided based on the determined category of content and the analysis result. The content transmitting unit may determine a target level corresponding to the determined category based on the analysis result, and the content transmitting unit may acquire the content to be provided based on the determined target level.

The information processing system may further include an environmental information acquiring unit that acquires environmental information corresponding to the user. The content transmitting unit may acquire the content to be provided based on the environmental information and the analysis result.

The user interface may be configured to receive an action input by a user, and the life rhythm analyzing unit may analyze the user's life rhythm based on the action input by the user. The action input by the user may correspond to information indicating at least one of a wake-up time of the user, a bedtime of the user, a diet of the user, and a category of exercise by the user..

The content provided by the transmitting unit may be a Uniform Resource Locator (URL).

The information processing system may also include a mobile terminal that includes the user interface and the life rhythm analyzing unit; and a server that includes the content transmitting unit, wherein the mobile terminal further includes an interface that sends the received input to the server and receives the provided content from the server.

The information processing system may also include a mobile terminal that includes the user interface and a communication interface that sends the received input to a server; and the server that includes the life rhythm analyzing unit and the content transmitting unit and a communication interface that sends the provided content to the mobile terminal.

According to another example, the disclosure is directed to an information processing method performed by an information processing system, the method comprising: receiving, at a user interface, an input by a user; analyzing the user's life rhythm based on information collected by a sensor for the analysis; acquiring the input and analysis result of the analyzing; and providing content based on the input and the analysis result.

According to another example, the disclosure is directed to a non-transitory computer readable medium including computer program instructions, which when executed by an information processing system, causes the information processing system to perform a method comprising: receiving, at a user interface, an input by a user; analyzing the user's life rhythm based on information collected by a sensor for the analysis; acquiring the input and analysis result of the analyzing; and providing content based on the input and the analysis result.

### Advantageous Effects of Invention

According to this technique, in the terminal device, a request for content corresponding to a date and time input from a user is issued to the external device, and content that is selected and supplied based on the life rhythm of the user on the input date and time by the external device in response to the content request is presented. In the external device, in response to a content request of the user from the terminal device, content that matches the life rhythm of the user is selected from the content storing unit or the transmitted content storing unit, and is transmitted to the terminal device. The transmitted content storing unit stores content selected from the content storing unit in accordance with the life rhythm of the user, and the stored content is associated with dates and times. Accordingly, information that matches the life rhythm of the user at a desired time can be readily made available.

### Brief Description of Drawings

[fig.1] Fig. 1 is a block diagram showing the structure of an information processing system.
[fig.2] Fig. 2 is a diagram showing an example external view of a terminal device.
[fig.3] Figs. 3(A) through 3(C) are diagrams showing example information entry screens.
[fig.4] Figs. 4(A) and 4(B) show example channel select screens.
[fig.5] Figs. 5(A) and 5(B) show example date and time entry screens.
[fig.6] Fig. 6 is a flowchart showing operations of the terminal device.
[fig.7] Fig. 7 is a table showing example information stored in a collected information storing unit.
[fig.8] Fig. 8 is a table showing example results of action pattern analyses.
[fig.9] Fig. 9 is a table showing example results of exercise quantity pattern analyses.
[fig.10] Fig. 10 is a table showing example results of preference pattern analyses.
[fig.11] Fig. 11 is a table showing example results of specific daily pattern analyses.
[fig.12] Fig. 12 is a flowchart showing operations of a life rhythm analyzing unit.
[fig.13] Fig. 13 is a table showing an example of stored content information.
[fig.14] Fig. 14 is a table showing an example of stored environmental information.
[fig.15] Figs. 15(A) and 15(B) are tables for explaining an action determining operation.
[fig.16] Figs. 16(A) and 16(B) are tables for explaining a category determining operation.
[fig.17] Fig. 17 is a table for explaining a target level determining operation.
[fig.18] Fig. 18 is a flowchart showing operations of a content transmitting unit.
[fig.19] Fig. 19 is a flowchart showing an operation to be performed when the content corresponding to each hour of a day is to be acquired.
[fig.20] Fig. 20 is a diagram showing a displayed example of a content list.
[fig.21] Fig. 21 is a diagram illustrating a content presenting operation.

### Description of Examples

The following is a description of an exemplary mode for carrying out the present technique. The description will be given in the following order:
1. Structure of information processing system
2. Operations of terminal device
3. Operations of life rhythm analyzing unit
4. Operations of server device
5. Other operations of terminal device

### <1. Structure of Information Processing System>

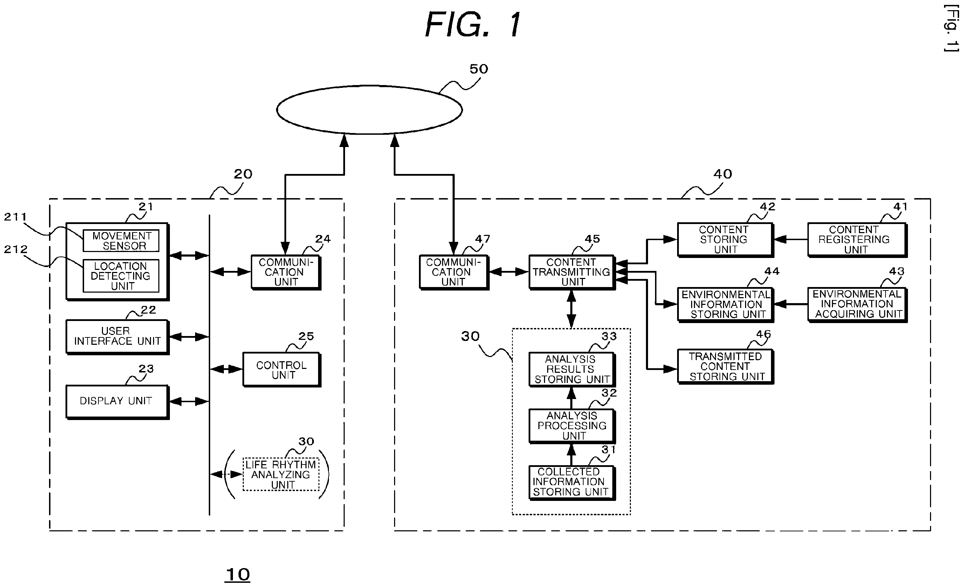

Fig. 1 is a block diagram showing the structure of an information processing system. An information processing system 10 is formed with a terminal device 20 and an external device such as a server device 40. The terminal device 20 and the server device 40 are connected via a network 50, for example.

The terminal device 20 includes an information collecting unit 21, a user interface unit 22, a display unit 23, a communication unit 24, and a control unit 25. The server device 40 includes a content registering unit 41, a content storing unit 42, an environmental information acquiring unit 43, an environmental information storing unit 44, a content transmitting unit 45, a transmitted content storing unit 46, and a communication unit 47. Further, the information processing system 10 has a life rhythm analyzing unit 30 provided in the terminal device 20 or in the server device 40. Fig. 1 shows an example case where the life rhythm analyzing unit is provided in the server device 40. Content is information in the form of images, characters, sound, control signals for a motor, and the like. In the example case described below, the content is information in the form of images and characters.

The information collecting unit 21 of the terminal device 20 collects information to be used for analyzing a life rhythm. For example, a movement sensor 211 having a pedometer function and the like is provided in the information collecting unit 21, to generate information related to movement of a user. Also, a location detecting unit 212 for acquiring location information may be provided in the information collecting unit 21. The location detecting unit 212 has a function to detect the actual location by receiving a positioning signal used in GPS (Global Positioning System), a function to detect the actual location by receiving a signal from a base station of a portable telephone device, a function to detect the actual location by acquiring the information on the location of a router based on the address contained in a signal received from the router in WiFi (wireless fidelity), and the like. The information collected by the information collecting unit 21 and information that is input through user information input operations as described later will be hereinafter referred to as collected information.

The user interface unit 22 is for a user to select from a menu, input information, and the like. The user interface unit 22 is formed with operating switches, a touch panel, and the like. The user interface unit 22 generates operating signals in accordance with user operations, and outputs the operating signals to the control unit 25.

The display unit 23 is formed with a liquid crystal display element and a flat display element such as organic EL. The display unit 23 displays the menu, information about content supplied from the server device 40, and the like.

The communication unit 24 performs communication with the server device 40. Under the control of the control unit 25, the communication unit 24 transmits collected information or a content request to the server device 40. The communication unit 24 also receives content supplied from the server device 40.

The control unit 25 controls the respective components of the terminal device 20, and controls the respective components to perform operations in accordance with user operations. For example, the control unit 25 provides collected information or issues a content request to the server device 40 in accordance with a user operation. Also, the control unit 25 uses user identification information in providing collected information or issuing a content request, so that the server device 40 can identify the user who has provided the collected information or made the content request. Further, if allowed by the user in advance, the control unit 25 provides collected information to the server device 40 on a regular basis.

The control unit 25 also reproduces the content supplied from the server device 40 in response to a content request, and causes the display unit 23 to display various kinds of information indicated by the content. In a case where the content is information in the form of images and characters, for example, the information is displayed by the display unit 23. In a case where the content indicates a URL (Uniform Resource Locator), the control unit 25 accesses the host indicated by the URL via the network 50, and acquires information. The information is then displayed by the display unit 23. When reproduction of content comes to an end, the control unit 25 issues a content request to the server device 40.

The life rhythm analyzing unit 30 includes a collected information storing unit 31, an analysis processing unit 32, and an analysis results storing unit 33. The collected information storing unit 31 stores collected information provided by the terminal device 20.

The analysis processing unit 32 analyzes the life pattern of a user, based on the collected information stored in the collected information storing unit 31. For example, the analysis processing unit 32 analyzes the pattern of a daily life rhythm or a weekly life rhythm, and supplies the analysis results to the analysis results storing unit 33.

The analysis results storing unit 33 stores life rhythm analysis results supplied from the analysis processing unit 32. In response to a request from the content transmitting unit 45, the analysis results storing unit 33 supplies stored analysis results to the content transmitting unit 45.

The content registering unit 41 of the server device 40 registers content to be provided to the terminal device 20. The content is information related to actions that form the life pattern of a user. The content registering unit 41 registers the content associated with attributes related to the actions. The registration of the content may be performed by content providers online, or may be performed off-line in accordance with instructions from the content providers.

The content storing unit 42 stores the content registered in association with the attributes related to the actions. The content stored in the content storing unit 42 is read by the content transmitting unit 45.

The environmental information acquiring unit 43 acquires environmental information related to the life of a user. The environmental information acquiring unit 43 acquires meteorological information such as temperature, daylight hours, precipitation, wind direction, wind velocity, as the environmental information, for example. In acquiring the meteorological information, the environmental information acquiring unit 43 acquires the information corresponding to the location of the user. The location of the user may be registered beforehand in the server device 40, or the location detected by the location detecting unit 212 of the information collecting unit 21 in the terminal device 20 may be used. Further, a future location of the user may be predicted, and the environmental information about the area including the predicted location may be acquired. A future location can be predicted by referring to the past action history of the user, for example.

The environmental information storing unit 44 stores the environmental information acquired by the environmental information acquiring unit 43. The environmental information storing unit 44 is read by the content transmitting unit 45.

Based on collected information supplied from the terminal device 20 to select the content that matches a life rhythm, the content transmitting unit 45 analyzes the life rhythm of a user. By further analyzing the action of the user on a designated date and time based on the analysis result, the content transmitting unit 45 selects the content related to the analysis results from the content storing unit 42, and transmits the content to the content requester. In a case where the life rhythm analyzing unit 30 that analyzes the life rhythm of a user is provided, the content transmitting unit 45 identifies the user who has made the content request, and reads the results of the analysis on the life rhythm of the identified user on the designated date and time from the analysis results storing unit 33 of the life rhythm analyzing unit 30. Based on the read analysis results, the content transmitting unit 45 analyses the actions of the user, and selects the content related to the analysis results from the content storing unit 42, and transmits the content to the content requester.

The transmitted content storing unit 46 associates the date and the time with the content selected by the content transmitting unit 45 from the content storing unit 42 in accordance with the results of the analysis on the action of the user. The transmitted content storing unit 46 then stores the selected content associated with the date and the time. Also, in response to a request from the content transmitting unit 45, the transmitted content storing unit 46 outputs stored content to the content transmitting unit 45.

The communication unit 47 communicates with the communication unit 24 of the terminal device 20. The communication unit 47 receives a content request or the like supplied from the terminal device 20, and supplies the content request or the like to the content transmitting unit 45. The communication unit 47 also transmits the content supplied from the content transmitting unit 45 in response to the content request, to the terminal device 20.

In the above description, the terminal device 20 displays content by using the display unit 23 as a content presenting unit. In a case where content is audio information, however, the content is presented through a speaker or the like. Also, the content storing unit 42 and the environmental information storing unit 44 may not be provided in the server device 40, but may be provided in an external device such as a data center.

### <2. Operations of Terminal Device>

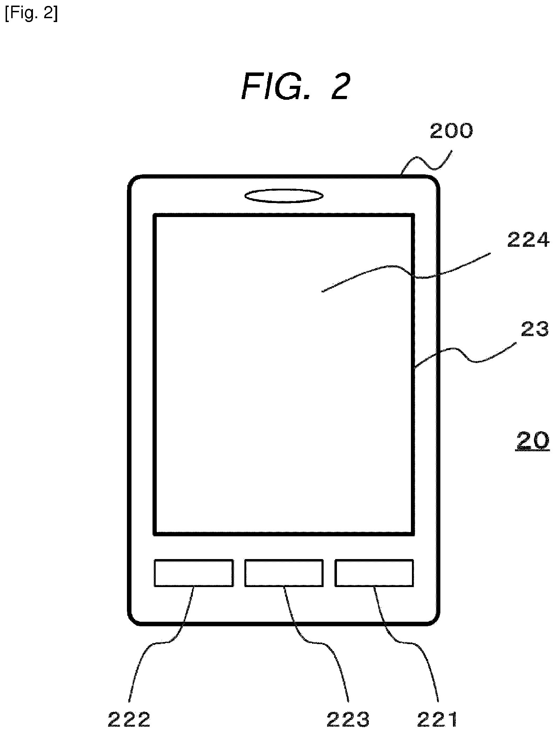

Fig. 2 shows an example external view of the terminal device 20. Operating switches 221, 222, and 223, which form the user interface unit 22, are provided on a housing 200 of the terminal device 20. A touch panel 224 forming the user interface unit 22 is formed on the display surface of the display unit 23. For example, the operating switch 221 is a switch for calling up an entry screen for information to be used to analyze a life rhythm. The operating switch 222 is a switch for calling up a channel select screen for designating a category of content to be acquired from the server device 40. The operating switch 223 is a switch for calling up a date and time entry screen for acquiring the content corresponding to a desired date and time.

The control unit 25 of the terminal device 20 displays an information entry screen on the display unit 23 in accordance with an operation of the operating switch 221. Further, when a user operation is performed on the display of the information entry screen, the control unit 25 determines what kind of input operation the user has performed, based on the operated spot and the displayed screen on the touch panel 224. The control unit 25 then performs an information input operation, based on the results of the determination.

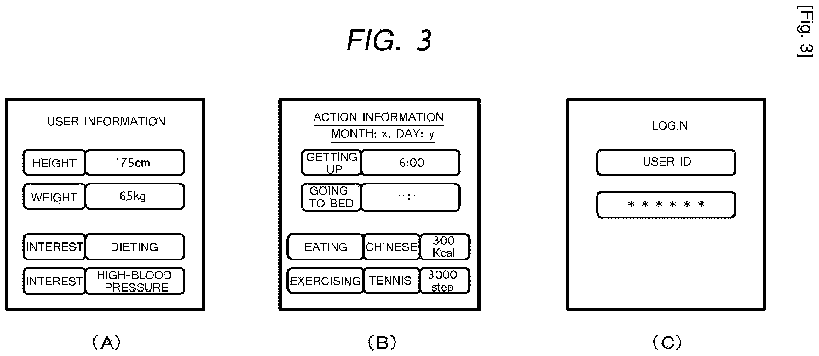

Figs. 3(A) through 3(C) show example information entry screens. Fig. 3(A) shows an example user information entry screen. From the user information entry screen, physical features of a user and information for selecting content of interest to the user can be input. For example, as shown in Fig. 3(A), there are text fields for inputting the height and weight as physical features, and text fields for inputting subjects of interest. Information is not necessarily input from the text fields. Instead, spin buttons may be provided to allow inputting of the height and weight, and list boxes or combo boxes may be provided to allow inputting of subjects of interest.

Fig. 3(B) shows an example action information entry screen. From the action information entry screen, action information that cannot be collected by the information collecting unit 21 can be input, and action information can be input by the user, instead of the information collecting unit 21. For example, as shown in Fig. 3(B), there are text fields for inputting wake-up time, bedtime, a meal category, the number of calories consumed, a category of exercise, and the number of steps. As described above, information is not necessarily input from the text fields. Instead, spin buttons may be provided to allow inputting of wake-up time, bedtime, and the number of steps, and list boxes or combo boxes may be provided to allow inputting of a category of meal and a category of exercise. In inputting the action information, time information is associated with the action information, so as to allow determination on what time and which action the action information is related to. As for the time information to be associated with the action information, the control unit 25 may automatically generate time information indicating the time at which information is input, and the user may be allowed to input time information.

Fig. 3(C) shows an example login screen. The login screen includes a text field for inputting the user ID for identifying the user.

The control unit 25 of the terminal device 20 displays a channel select screen on the display unit 23 in accordance with an operation of the operating switch 222. Further, when a user operation is performed on the display of the channel select screen, the control unit 25 determines what kind of channel select operation the user has performed, based on the operated spot and the displayed screen on the touch panel 224.

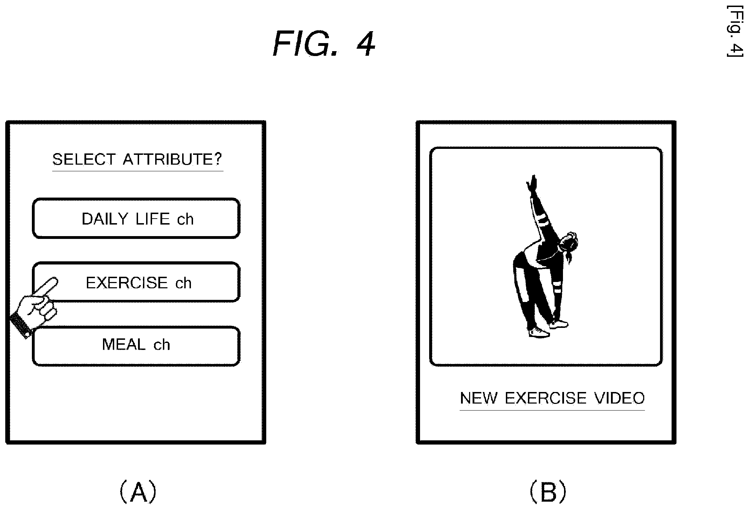

Figs. 4(A) and 4(B) show example channel select screens. From a channel select screen, one of channels can be selected. In Fig. 4(A), for example, a daily life channel, an exercise channel, or a meal channel can be selected. The daily life channel is a channel that provides timely meal, exercise, sleep information that matches the life rhythm of the user. The exercise channel is a channel that provides timely exercise-related information based on exercise history information as to the user, in synchronization with the life rhythm of the user. The meal channel is a channel that provides timely meal-related information that matches the users preference or the like, in synchronization with the life rhythm of the user.

The control unit 25 of the terminal device 20 displays a date and time entry screen on the display unit 23 in accordance with an operation of the operating switch 223. Further, when a user operation is performed on the display of the date and time entry screen, the control unit 25 determines what kind of date and time input operation the user has performed, based on the operated spot and the displayed screen on the touch panel 224. The control unit 25 then performs a date and time input operation, based on the results of the determination.

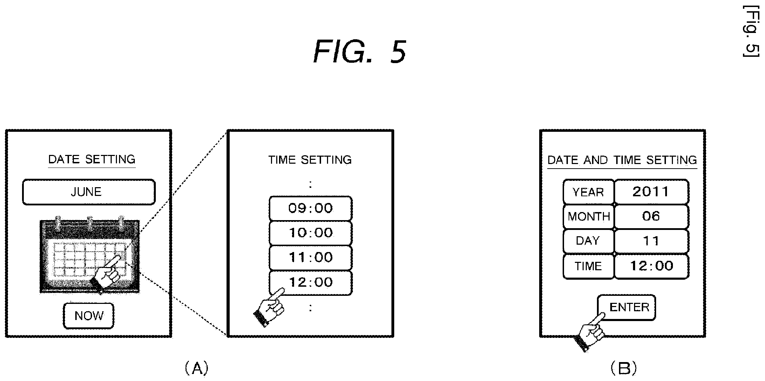

Figs. 5(A) and 5(B) show example date and time entry screens. Fig. 5(A) shows a date and time entry screen for designating a date by using a calendar, and designating a time after the designation of the date. Fig. 5(B) shows a date and time entry screen that includes date and time entry text fields from which a year, a month, a day, and a time are input. As described above, a date and a time are not necessarily input from the text fields, and spin buttons may be provided to allow the inputting.

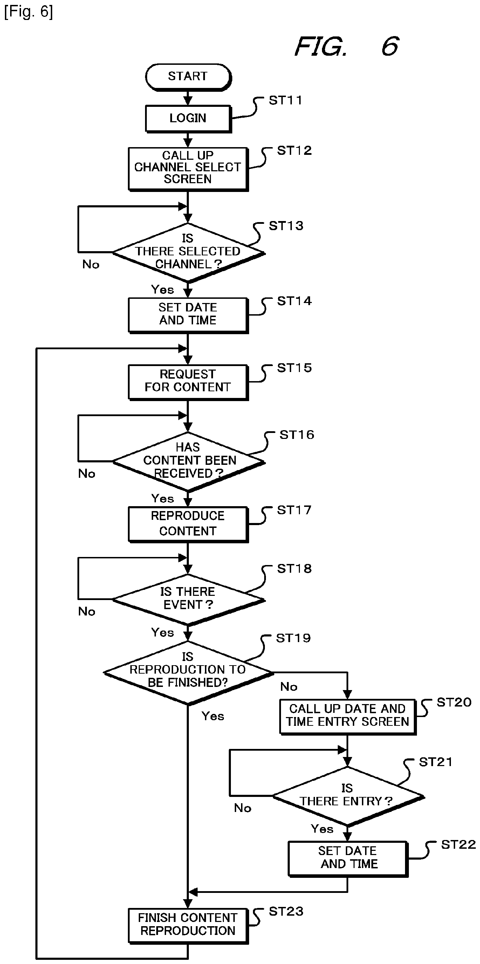

Fig. 6 is a flowchart showing operations of the terminal device. In step ST11, the control unit 25 logs on. The control unit 25 causes the display unit 23 to display the login screen, to receive an input of a user ID. The control unit 25 also transmits the input user ID to the server device 40. If there is a notification from the server device 40 that the user has been confirmed, the process moves on to step ST12. If there is a notification that the user cannot be confirmed, the display unit 23 shows an indication to that effect.

In step ST12, the control unit 25 calls up the channel select screen. The control unit 25 controls the display unit 23 to display the channel select screen, and the process moves on to step ST13.

In step ST13, the control unit 25 determines whether a channel has been selected. If a selected channel is not detected, the process returns to step ST13. If a selected channel is detected, the process moves on to step ST14.

In step ST14, the control unit 25 performs date and time setting. The control unit 25 controls the display unit 23 to display the date and time entry screen. Further, the control unit 25 sets the date and time input by the user as a designated date and time for content acquirement, and the process moves on to step ST15.

In step ST15, the control unit 25 issues a content request. The control unit 25 requests the server device 40 for content corresponding to the designated date and time, and the process moves on to step ST16.

In step ST16, the control unit 25 determines whether the content has been received. Unless the content is supplied from the server device 40 in response to the content request, the process returns to step ST16. Upon receipt of the content from the server device 40, the process moves on to step ST17.

In step ST17, the control unit 25 reproduces the content. The control unit 25 reproduces the received content, and displays the information in the content on the screen of the display unit 23. The process then moves on to step ST18.

In step ST18, the control unit 25 determines whether an event has occurred. In a case where an event has not occurred, for example in a case where a user operation has not been performed, the process returns to step ST18. In a case where a user operation has been detected, the process moves on to step ST19.

In step ST19, the control unit 25 determines whether the event is an end of reproduction or a switch operation. In a case where the touch panel of the display unit 23 has been operated, for example, the control unit 25 determines that the reproduction is to be finished, and the process moves on to step ST23. In a case where a switch operation has been performed with the operating switch 223, the process moves on to step ST20.

In step ST20, the control unit 25 calls up the date and time entry screen. The control unit 25 controls the display unit 23 to display the date and time entry screen, and the process moves on to step ST21.

In step ST21, the control unit 25 determines whether a date and a time have been input. If inputting of information has not been detected, the process returns to step ST21. If inputting of a date and a time has been detected, the process moves on to step ST22.

In step ST22, the control unit 25 performs date and time setting. The control unit 25 sets the input date and time as a designated date and time, and the process moves on to step ST23.

Moving on to step ST23 from step ST19 or ST22, the control unit 25 ends the reproduction of the content, and the process returns to step ST15.

In this manner, the terminal device 20 sets a designated date and time, and requests the server device 40 for the content.

Although not shown in Fig. 6, in a case where a switch operation has been performed with the operating switch 221, the information entry screen is displayed to allow inputting of information. In a case where a switch operation has been performed with the operating switch 222, the channel select operation screen is displayed, and an operation is performed to request content on a channel selected by the user.

As described above, the terminal device 20 provides the server device 40 with collected information for analyzing a life rhythm. Accordingly, after analyzing the life rhythm in a later described manner using the provided collected information, the server device 40 can provide the content in accordance with the results of a prediction of the user's action on the designated date and time.

### <3. Operations of the Life Rhythm Analyzing Unit>

The life rhythm analyzing unit 30 analyzes the life rhythms of users, based on collected information.

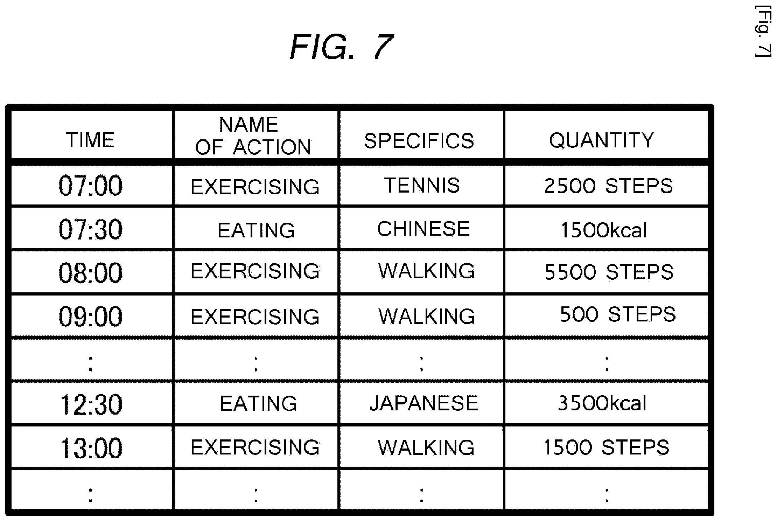

The collected information storing unit 31 stores information input from the information entry screen and collected information acquired by the information collecting unit 21 as the history about each user. Fig. 7 shows example collected information stored in the collected information storing unit 31. For example, the collected information is stored, with the names of actions, the categories, and the quantities being associated with one another. In the "name of action" column, actions are classified into exercising and eating. In the "category" column, the categories of exercises and categories of meals are shown. Further, the "quantity" column shows the numbers of steps taken in exercises, and the numbers of calories taken at mealtimes.

The analysis processing unit 32 analyzes a life rhythm, using the collected information stored in the collected information storing unit 31. Based on the collected information stored in the collected information storing unit 31, the analysis processing unit 32 analyzes an action pattern, an action-related quantity pattern such as an exercise quantity pattern, a preference pattern, and a specific daily pattern.

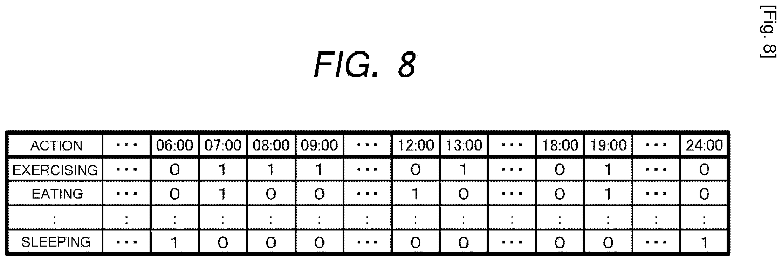

Fig. 8 shows example results of action pattern analyses. The results of the action pattern analysis show which action is taken most often among the respective actions in chronological order. For example, the analysis processing unit 32 compares the stored exercise quantity or the stored consumed calories information with a predetermined threshold value, and, based on the comparison result, determines whether an exercise or a meal has been taken. If the exercise quantity is equal to or greater than the threshold value, the analysis processing unit 32 determines that an exercise has been taken, and sets the analysis result to "1". Likewise, if the number of consumed calories is equal to or larger than the predetermined threshold value, the analysis processing unit 32 determines that a meal has been taken, and sets the analysis result to "1". In a case where the analysis processing unit 32 determines that any exercise or meal has not been taken, the analysis processing unit 32 sets the analysis result to "0". Further, the analysis processing unit 32 sets the initial value of each action pattern analysis result to "0", and combines the analysis results of each day in a time-synchronized manner, to obtain frequency values. In this manner, the analysis processing unit 32 generates the action pattern analysis results. Generated in this manner, the action pattern analysis results serve as the model of an action pattern. Therefore, by using the action pattern analysis results, it is possible to determine which action is often taken at what time based on the frequency values in the action pattern analysis results.

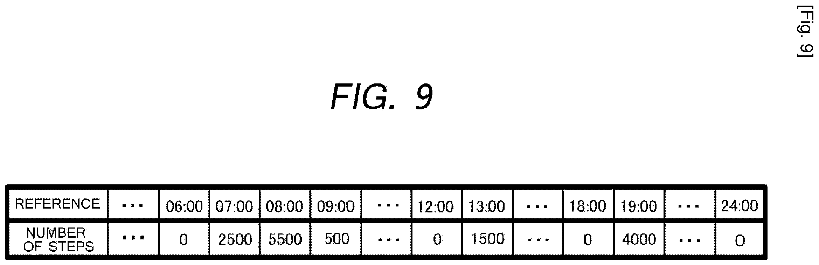

Fig. 9 shows example results of exercise quantity pattern analyses. The analysis processing unit 32 calculates the statistical values such as the average values of the hourly exercise quantities of each day, to obtain exercise quantity pattern analysis results. Generated in this manner, the exercise quantity pattern analysis results serve as the model of expected exercise quantities.

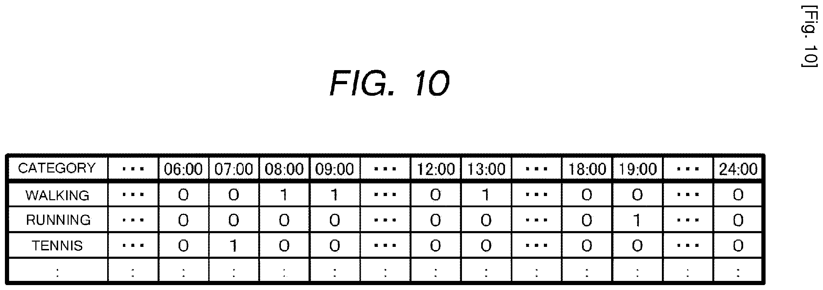

Fig. 10 shows example results of preference pattern analyses. Specifically, Fig. 10 shows the results of exercise category preference pattern analyses. The preference pattern analysis results show which category of exercise is most often taken in chronological order. For example, based on the stored "category" information, the analysis processing unit 32 analyzes which category of exercise has been taken, and sets the result of the analysis as to the category of the taken exercise to "1". The analysis processing unit 32 also sets the result of the analysis as to each category of exercise that has not been taken to "0". Further, the analysis processing unit 32 puts the initial value of each preference pattern analysis result to "0" like each action pattern analysis result. The analysis processing unit 32 then combines the results of analyses of each day in a time-synchronized manner, to obtain frequency values. In this manner, the analysis processing unit 32 generates the preference pattern analysis results. Generated in this manner, the preference pattern analysis results serve as the model of an exercise category preference. Therefore, by using the preference pattern analysis results, it is possible to determine which category of exercise is often taken at what time based on the frequency values in the preference pattern analysis results.

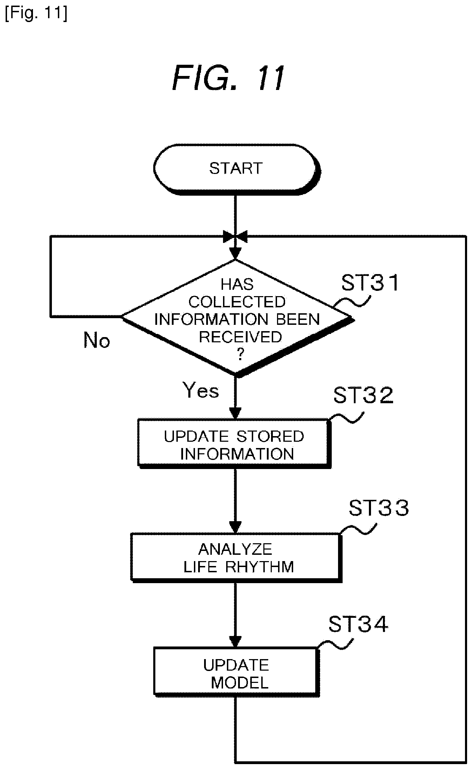

A life rhythm varies between weekdays and holidays, for example. Therefore, not only a life rhythm analysis is carried out in the same manner on a daily basis, but also a life rhythm analysis may be carried out in a different manner on each day of the week. In the life rhythm analysis on each day of the week, the collected information is classified into the days of the week, and the life rhythm is analyzed by using the collected information about the day of the week on which the life rhythm analysis is being analyzed. Fig. 11 shows example results of specific daily pattern analyses. The specific daily pattern analysis results include the results of pattern analyses that are carried out in the same manner each day of the week, and the results of pattern analyses that are carried out in a different manner on each day of the week. That is, the specific daily pattern analysis results consist of eight analysis result patterns. For example, the "(quantity) walking" of each day of the week is associated with the hourly distribution data that corresponds to each date and is shown in Fig. 9. Also, the "(preference) exercise category" of each day is associated with the hourly distribution data that corresponds to each date and is shown in Fig. 10. Likewise, the action pattern distribution shown in Fig. 8 is associated with each corresponding date, and is managed in the table shown in Fig. 11.

The analysis results storing unit 33 stores the models related to the life rhythm analyzed by the analysis processing unit 32. The models related to the life rhythm include the action pattern analysis results, the quantity pattern analysis results, and the preference pattern analysis results, and the specific daily pattern analysis results. The models related to the life rhythm stored in the analysis results storing unit 33 are updated with new analysis results, when the collected information stored in the collected information storing unit 31 is updated, and a life rhythm analysis is carried out by the analysis processing unit 32.

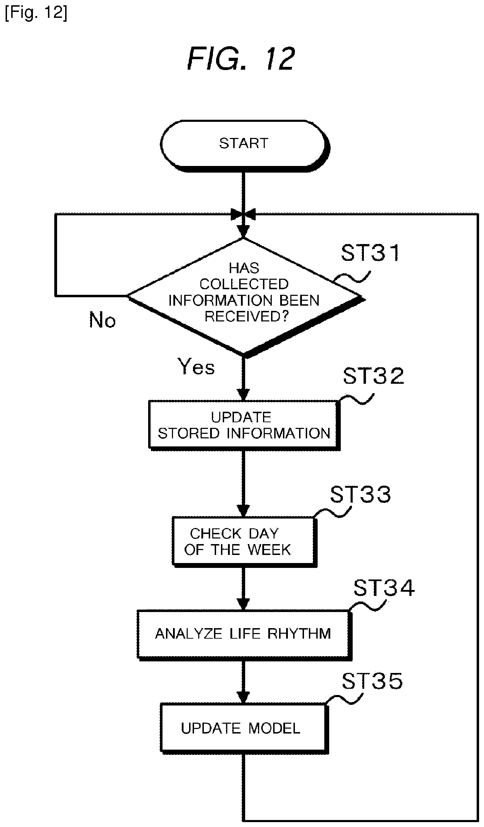

Fig. 12 is a flowchart showing operations to be performed by the life rhythm analyzing unit 30 in a case where the life rhythm analyzing unit 30 is provided in the server device 40.

In step ST31, the life rhythm analyzing unit 30 determines whether collected information has been received. Where information input from the information entry screen or information acquired by the information collecting unit 21 has been supplied from the terminal device 20, the process moves on to step ST32. Where collected information has not been supplied, the process returns to step ST31.

In step ST32, the life rhythm analyzing unit 30 updates the stored information. The collected information storing unit 31 of the life rhythm analyzing unit 30 stores the newly supplied collected information as a history, and the process moves on to step ST33.

In step ST33, the life rhythm analyzing unit 30 confirms the day of the week. The analysis processing unit 32 confirms to which day of the week the collected information corresponds, and the process moves on to step ST34.

In step ST34, the life rhythm analyzing unit 30 carries out a life rhythm analysis. As the collected information stored in the collected information storing unit 31 has been updated, the analysis processing unit 32 of the life rhythm analyzing unit 30 carries out the life rhythm analysis, using the updated information corresponding to the day on which the update has been performed. The process then moves on to step ST35. In the life rhythm analysis, the daily pattern that is the same on each day of the week, and the pattern of the day on which the update has been performed are analyzed.

In step ST35, the life rhythm analyzing unit 30 updates the models related to the life rhythm. Based on the analysis results of step ST34, the analysis results storing unit 33 of the life rhythm analyzing unit 30 updates the stored models related to the life rhythm, and the process returns to step ST31.

As described above, the life rhythm analyzing unit 30 analyzes the life rhythm of a user, based on the collected information. The life rhythm analyzing unit 30 then generates an action pattern, a preference pattern, and the like. Therefore, by using the pattern analysis results, it is possible to predict actions and the like of the user on a designated date and time.

### <4. Operations of the Server Device>

The content information registered by the content registering unit 41 of the server device 40 is stored into the content storing unit 42.

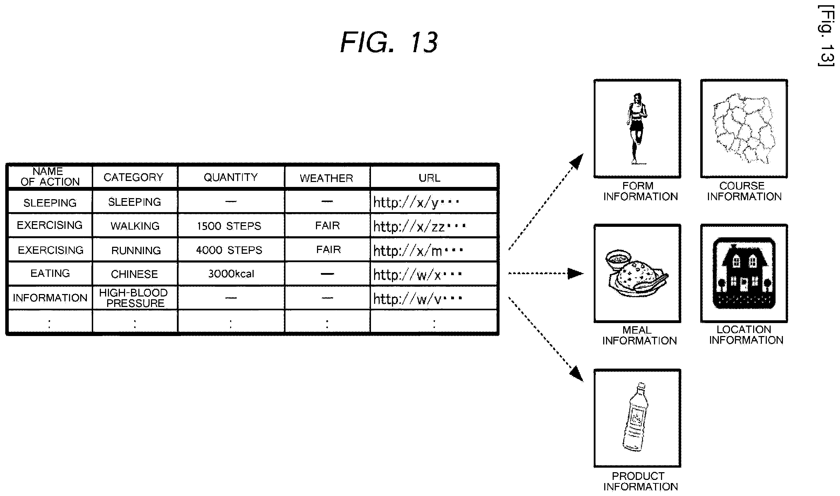

Fig. 13 shows example content information stored in the content storing unit 42. The content information contains the metadata indicating content attributes, as well as the specifics of the content (such as URLs (Uniform Resource Locators)). The metadata is the information to be used for selecting content in accordance with the life rhythm or the like of the user, and is registered in accordance with the content attributes by the content provider, for example. As the metadata, information about the names, categories, and quantities of the actions used in the life rhythm analysis, and environmental information are used.

The environmental information acquired by the environmental information acquiring unit 43 of the server device 40 is stored into the environmental information storing unit 44.

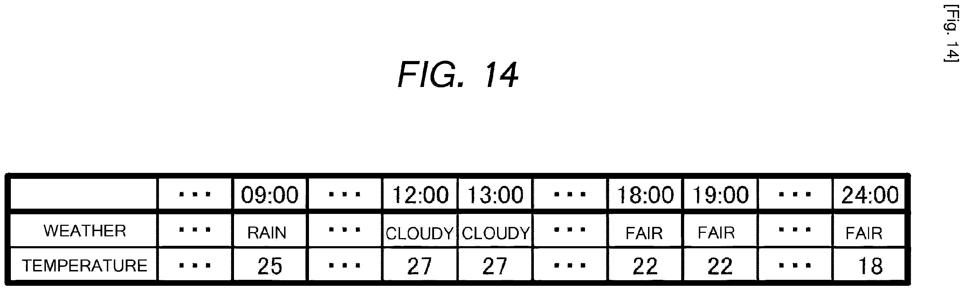

Fig. 14 shows example environmental information stored in the environmental information storing unit 44. As the environmental information, information on weather, temperature, and the like is acquired. The environmental information is divided into regions and is associated with hours, so that information can be acquired in accordance with the location of the content user. In Fig. 14, the environmental information as to one region is shown as an example.

The content transmitting unit 45 of the server device 40 determines at each hour the parameter for searching the registered content, based on the respective models indicating a life rhythm and the location information and channel information contained in a request from a client. Further, the content transmitting unit 45 selects content in accordance with the determined parameter, and transmits the selected content to the client.

In a case where the daily life channel is selected in the terminal device 20, the content transmitting unit 45 calculates the action probabilities, taking into account the frequencies at the designated hour in the action pattern analysis results. Further, the content transmitting unit 45 conducts a drawing based on the probabilities, and determines an action. In a case where a specific channel such as the meal channel or the exercise channel is selected in the terminal device 20, the content transmitting unit 45 selects content by picking up the action of the selected channel. Therefore, the content transmitting unit 45 does not conduct a drawing based on the probabilities.

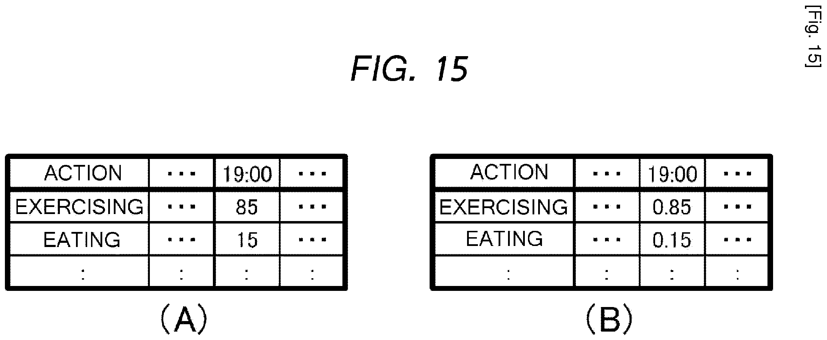

Figs. 15(A) and 15(B) are tables for explaining the action determining operation. For example, in a case where the frequency value of exercising is "85" while the frequency value of eating is "15" between 18:00 and 19:00 in the action pattern analysis results as shown in Fig. 15(A), the action probabilities are as follows: the probability of exercising is "0.85", and the probability of eating is "0.15", as shown in Fig. 15(B). A drawing is conducted based on the probabilities, and an action is determined.

In relation to the determined action, the content transmitting unit 45 then determines a category of content to be provided based on the preference pattern, and determines a target level of the value for selecting the content to be provided based on the quantity pattern.

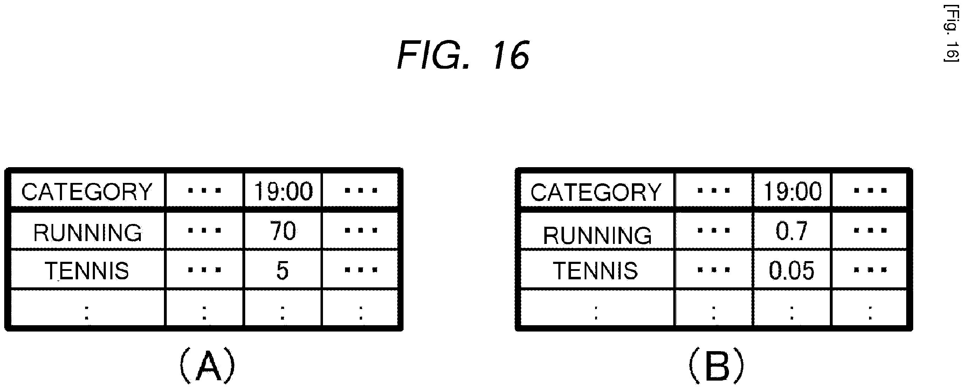

Figs. 16(A) and 16(B) are tables for explaining the category determining operation in a case where exercising is determined as an action. For example, in a case where the frequency value of running is "70" while the frequency value of tennis is "5" between 18:00 and 19:00 in the exercise preference pattern analysis results as shown in Fig. 16(A), the category probabilities are as follows: the probability of running is "0.70", and the probability of tennis is "0.05", as shown in Fig. 16(B). A drawing is conducted based on the probabilities, and a category is determined.

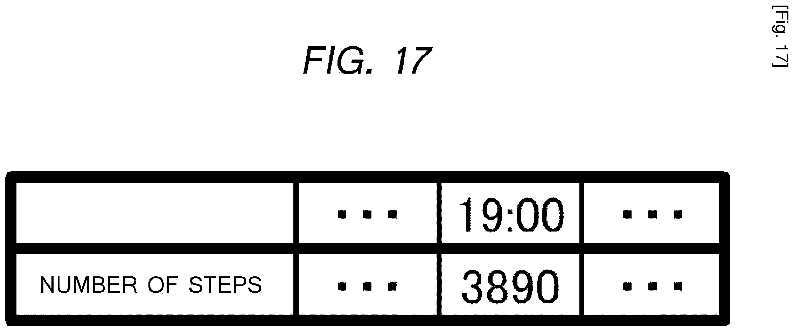

Fig. 17 is a table for explaining the target quantity level determining operation in a case where exercising is determined as an action. For example, in a case where the exercise quantity between 18:00 and 19:00 in the quantity pattern analysis results is "3890" as shown in Fig. 17, the target level is determined as "3890 steps".

Although Figs. 16 and 17 show a case where exercising is determined as an action, a meal category such as Chinese or Japanese is determined based on the preference pattern analysis results in a case where eating is determined as an action. Also, the number of calories to be consumed is determined as the quantity reference value. In the case of sleeping, the preference pattern consists of sleeping and getting up, and therefore, any quantity parameter is not used.

The content transmitting unit 45 also performs an environmental information determining operation. In determining an environment, environmental information is determined based on a designated date and time. The content transmitting unit 45 may determine environmental information corresponding to the designated date and time, based on the environmental information as to the area including the location indicated by location information supplied from the terminal device 20.

Further, in a case where a content request with a designated date and time has been issued, the content transmitting unit 45 determines a category of content to be provided, based on the action on the designated date and time and the preference pattern.

As described above, the content transmitting unit 45 determines an action, a category, a target level, a designated date and time, and the like, which are to be used as search parameters. For example, in a case where the action is exercising, the category of action is running, and the target level is 3890 steps on the designated date and time, those subjects and value are used as the search parameters. Further, the content transmitting unit 45 selects transmission candidate content from the content stored in the content storing unit 42, based on the search parameters. For example, the content having the metadata indicating exercising as the action and running as the category of action is searched, and the content with quantity meta close to the target level of 3890 steps is selected from the searched content and is set as the transmission candidates.

Further, the content transmitting unit 45 randomly selects a piece of content from pieces of content selected as transmission candidates, and sets the selected piece of content as content to be transmitted. The content transmitting unit 45 transmits the to-be-transmitted content to the terminal device 20. If a content request is issued from the terminal device 20 two or more times in the same time slot when a piece of content is selected and transmitted based on the search parameters, the same content is repeatedly provided. To provide new content, an operation to change the search parameters needs to be performed. However, the content transmitting unit 45 selects pieces of content as transmission candidates based on the search parameters. The content transmitting unit 45 then randomly selects content from the transmission candidates, and transmits the selected content. Accordingly, different content can be provided even in a case where a content request is issued from the terminal device two or more times in the same time slot.

Also, there are hours at which an action might not belong to any action category in the action pattern analysis results. In that case, a keyword that is input by the user is used as the search parameter in selecting transmission candidates. Specifically, keywords registered from the "interest" field in the user information entry screen are stored in the life rhythm analyzing unit 30, and content is selected by using a search parameter that is a keyword randomly selected from the stored keywords. For example, if an action does not belong to any action category in the action pattern analysis results, the action-related search parameter is set as "information". In a case where the randomly selected keyword is "high-blood pressure", pieces of content having "information" as the action and "high-blood pressure" as the category are selected as transmission candidates from the registered content.

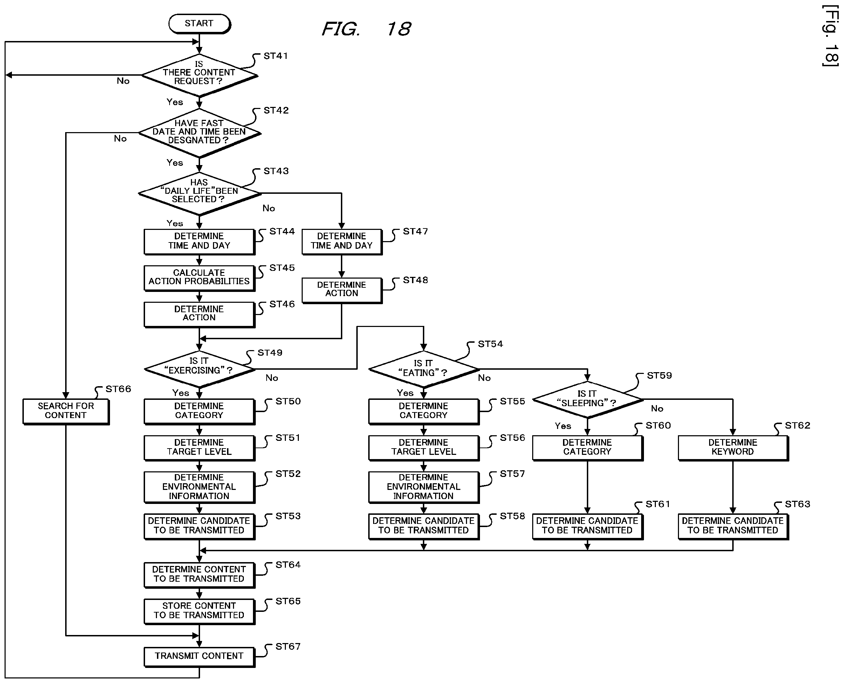

Fig. 18 is a flowchart showing operations to be performed by the content transmitting unit 45 when a content request with a designated date and time is issued.

In step ST41, the content transmitting unit 45 determines whether a content request has been issued. In a case where any content request has not been issued, the process returns to step ST41. In a case where a content request has been issued, the process moves on to step ST42.

In step ST42, the content transmitting unit 45 determines whether the designated date and time indicate past time. If the designated date and time does not indicate past time, the process moves on to step ST43. If the designated date and time indicates past time, the process moves on to step ST66.

In step ST43, the content transmitting unit 45 determines whether the selected channel is the daily life channel. If the selected channel is the daily life channel, the process moves on to step ST44. If the selected channel is some other channel, the process moves on to step ST47.

In step ST44, the content transmitting unit 45 determines a time and a day of the week. The content transmitting unit 45 determines a day of the week and a time based on the designated date and time, and the process moves on to step ST45.

In step ST45, the content transmitting unit 45 calculates action probabilities. Based on the frequency values on the determined day of the week and time in the action pattern analysis results, the content transmitting unit 45 calculates the probability of each action, and the process moves on to step ST46.

In step ST46, the content transmitting unit 45 determines an action. The content transmitting unit 45 conducts a drawing in accordance with the action probabilities, and determines an action. The process then moves on to step ST49.

In step ST47, the content transmitting unit 45 determines a time and a day of the week. The content transmitting unit 45 determines a day of the week and a time based on the designated date and time, and the process moves on to step ST48.

In step ST48, the content transmitting unit 45 determines an action. The content transmitting unit 45 picks up the action of the selected channel, and the process moves on to step ST49.

In step ST49, the content transmitting unit 45 determines whether the determined action is exercising. If the determined action is exercising, the process moves on to step ST50. If the determined action is not exercising, the process moves on to step ST54.

In step ST50, the content transmitting unit 45 determines a category. Based on the frequency values on the designated date and time in the exercise preference pattern analysis results, the content transmitting unit 45 calculates the probabilities of respective categories. Further, the content transmitting unit 45 conducts a drawing in accordance with the category probabilities, and determines a category of exercise. The process then moves on to step ST51.

In step ST51, the content transmitting unit 45 determines a target level. The content transmitting unit 45 determines the target level that is the exercise quantity on the designated date and time in the quantity pattern analysis results, and then the process moves on to step ST52.

In step ST52, the content transmitting unit 45 determines environmental information. The content transmitting unit 45 selects, from the information stored in the environmental information storing unit 44, the information corresponding to the location indicated by location information supplied from the terminal device 20 and to the determined time. In this manner, the content transmitting unit 45 determines the environmental information and the process moves on to step ST53.

In step ST53, the content transmitting unit 45 determines transmission candidates. The content transmitting unit 45 sets the action, the category, and the like determined in step ST46 or ST48 and steps ST50 through ST52 as the search parameters. Based on the search parameters, the content transmitting unit 45 selects transmission candidate content from the content stored in the content storing unit 42, and the process then moves on to step ST64.

Moving from step ST49 to step ST54, the content transmitting unit 45 determines whether the determined action is eating. If the determined action is eating, the process moves on to step ST55. If the determined action is not eating, the process moves on to step ST59.

In step ST55, the content transmitting unit 45 determines a category. Based on the frequency values at the determined time in the results of the preference pattern analysis on meal categories, the content transmitting unit 45 calculates the probabilities of the respective categories. Further, the content transmitting unit 45 conducts a drawing in accordance with the probabilities of the respective categories, and then the process moves on to step ST56.

In step ST56, the content transmitting unit 45 determines a target level. The content transmitting unit 45 determines the target level from the consumed calories on the designated date and time in the quantity pattern analysis results, and the process moves on to step ST57.

In step ST57, the content transmitting unit 45 determines environmental information. As in step ST52, the content transmitting unit 45 selects, from the information stored in the environmental information storing unit 44, the information corresponding to the location indicated by location information supplied from the terminal device 20 and to the determined time. In this manner, the content transmitting unit 45 determines the environmental information and the process moves on to step ST58.

In step ST58, the content transmitting unit 45 determines transmission candidates. The content transmitting unit 45 sets the action, the category, and the like determined in step ST46 or ST48 and ST55 through ST57 as the search parameters. Based on the search parameters, the content transmitting unit 45 selects transmission candidate content from the content stored in the content storing unit 42, and the process then moves on to step ST64.

Moving from step ST54 to step ST59, the content transmitting unit 45 determines whether the determined action is sleeping. If the determined action is sleeping, the process moves on to step ST60. If the determined action is not sleeping, the process moves on to step ST62.

In step ST60, the content transmitting unit 45 determines a category. The content transmitting unit 45 determines the category as sleeping, and the process moves on to step ST61.

In step ST61, the content transmitting unit 45 determines transmission candidates. The content transmitting unit 45 sets the action and the category determined in step ST46, ST48 and step ST60 as the search parameters. Based on the search parameters, the content transmitting unit 45 selects transmission candidate content from the content stored in the content storing unit 42, and the process then moves on to step ST64.

Moving from step ST59 to step ST62, the content transmitting unit 45 determines a keyword. The content transmitting unit 45 randomly selects the keyword from the keywords registered by the user, and the process moves on to step ST63.

In step ST63, the content transmitting unit 45 determines transmission candidates. The content transmitting unit 45 sets the keyword determined in step ST62 as the search parameter. Based on the search parameter, the content transmitting unit 45 selects transmission candidate content from the content stored in the content storing unit 42, and the process then moves on to step ST64.

In step ST64, the content transmitting unit 45 determines content to be transmitted. The content transmitting unit 45 randomly selects a piece of content from pieces of content selected as the transmission candidates, and determines the selected piece of content as the content to be transmitted. The process then moves on to step ST65.

In step ST65, the content transmitting unit 45 have the to-be-transmitted content stored. The content transmitting unit 45 associates the determined to-be-transmitted content with a date and a time, and stores the determined to-be-transmitted content and the date and time into the transmitted content storing unit 46. The process then moves on to step ST67.

Moving from step ST42 to step ST66, the content transmitting unit 45 searches for content. The content transmitting unit 45 searches the content stored in the transmitted content storing unit 46 for the content corresponding to the designated date and time. If there is no content corresponding to the designated date and time, content indicating that there is no corresponding content is searched for. The content transmitting unit 45 reads the content corresponding to the designated date and time, and transmits the content as the content to be transmitted. The process then moves on to step ST67.

In step ST67, the content transmitting unit 45 transmits the content. The content transmitting unit 45 transmits the to-be-transmitted content to the terminal device 20 that has issued the content request, and the process returns to step ST41.

As described above, the content transmitting unit 45 determines the search parameters that are an action, a category, a target level, environmental information, and the like corresponding to a designated date and time, from the results of analyses on the user's life rhythm pattern analyzed by the life rhythm analyzing unit 30. Using the search parameters, the content transmitting unit 45 selects transmission candidate content, and determines content to be transmitted among the transmission candidates. The content transmitting unit 45 then transmits the content to the terminal device 20. Accordingly, content in accordance with the result of a prediction of the user's action on a designated date and time can be provided.

### <5. Other Operations of the Terminal Device>

Further, in a case where a content request contains a date designated from the date and time entry screen, content corresponding to the respective hours of the day is acquired, and which content is to be provided is displayed at the time of time setting. In this manner, content can be intuitively selected.

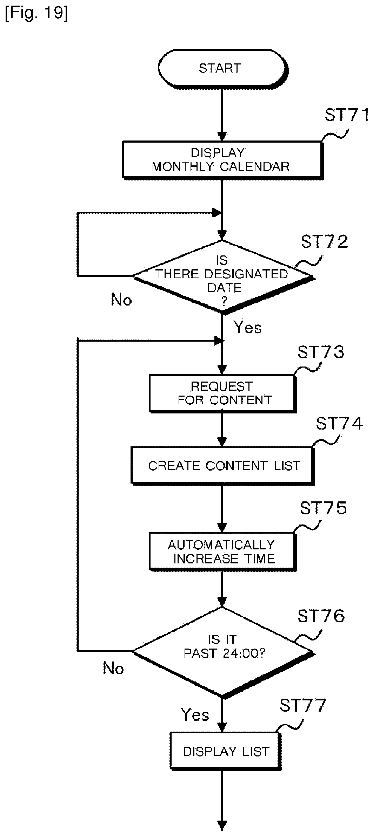

Fig. 19 is a flowchart showing an operation to be performed to acquire content corresponding to the respective hours of the day.

In step ST71, the control unit 25 displays a monthly calendar. The control unit 25 controls the display unit 23 to display the monthly calendar, and the process moves on to step ST72.

In step ST72, the control unit 25 determines whether a date has been designated. In a case where a date has not been designated, the process returns to step ST72. In a case where a date has been designated, the process moves on to step ST73.

In step ST73, the control unit 25 issues a content request. The control unit 25 designates a date and a time, and issues the content request to the server device 40. The process then moves on to step ST74.

In step ST74, the control unit 25 creates a content list. Using the content supplied from the server device 40, the control unit 25 creates the list in which the content is associated with the time, and the process moves on to step ST75.

In step ST75, the control unit 25 automatically increases the time. The control unit 25 increases the time, and the process then moves on to step ST76.

In step ST76, the control unit 25 determines whether the time is past 24:00. If the increased time is not past 24:00 of the day, the process returns to step ST73, and the control unit 25 issues a request for content corresponding to the increased time. If the increased time is past 24:00, the process moves on to step ST77.

In step ST77, the control unit 25 displays the list. The control unit 25 displays the list in which content corresponding to the respective hours of the designated date is shown.

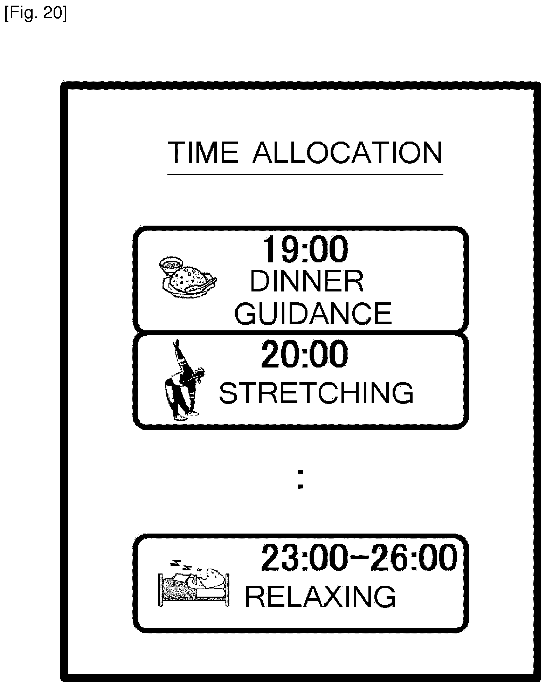

Through the above procedures, the list of the content corresponding to a day designated on the calendar can be created. Fig. 20 shows a displayed example of the content list. When a date is determined, a list of the content corresponding to the respective hours of the day is displayed. Accordingly, time setting can be intuitively performed by referring to the displayed list.

In a case where only a date is designated in a content request from the terminal device 20, the content transmitting unit 45 of the server device 40 automatically updates the time, using a clock provided in the server device. Content may be selected and transmitted at each hour, based on the life rhythm of the user on the designated date. In this manner, the terminal device 20 can display the content list only by designating a date, without constantly updating the time from the terminal device 20 and issuing a content request. Alternatively, the control unit 25 of the terminal device 20 may have the function to automatically update the time.

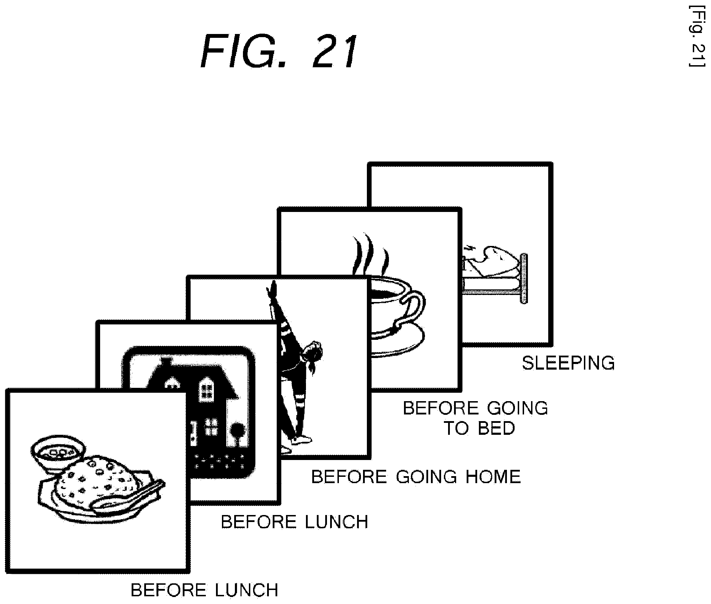

As described above, in the information processing system 10, client-server functions, registered content, and respective models indicating life rhythms analyzed based on logs of respective users are used, and a date and a time are designated. In this manner, content in accordance with the results of a prediction of a user's action on a designated date and time can be provided. A content user can receive content that matches his/her life rhythm by designating a date and a time and making a content request. Accordingly, use of the information in content is facilitated. For example, as shown in Fig. 21, a user can easily obtain and use information related to meals or restaurants before lunch, exercise information before going home, information related to relaxing items before going to bed, and relaxing music or environmental sound while in bed. Further, content is selected by combining parameters such as a category and a quantity. Accordingly, content that is new to content users can be provided. Also, a content user can easily obtain personalized content in synchronization with his/her life rhythm simply by selecting a channel that indicates an attribute. Further, the content provider only has to set metadata about content and then register the content to efficiently acquire information only to users interested in the content through the information processing system 10.

In the above described example, the life rhythm analyzing unit 30 is provided in the server device 40. However, the life rhythm analyzing unit 30 may be provided in the terminal device 20. In a case where the life rhythm analyzing unit 30 is provided in the server device 40, the terminal device 20 only has to provide the server device 40 with collected information to have a life rhythm pattern analyzed. Accordingly, the processing in the terminal device 20 can be reduced. In a case where the life rhythm analyzing unit 30 is provided in the terminal device 20, the terminal device 20 does not provide the collected information, but adds search parameters for selecting content that matches the life rhythm at a designated time to a content request. The display unit 23 displays content that is selected and supplied in accordance with the life rhythm by the server device 40 based on the search parameters added to the content request.

The series of operations described in the specification can be performed by hardware, software, or a combination of hardware and software. In a case where the operations are performed by software, the programs in which the operation sequences are written are installed on a memory incorporated into dedicated hardware in a computer, and are then executed. Alternatively, the programs are installed on a general-purpose computer that can perform various kinds of operations, and are then executed.

The programs can be recorded beforehand on a hard disk or a ROM (Read Only Memory) as a recording medium, for example. Alternatively, the programs can be temporarily or permanently stored (recorded) in a removable recording medium such as a flexible disk, a CD-ROM (Compact Disc Read Only Memory), a MO (Magneto Optical) disk, a DVD (Digital Versatile Disc), a magnetic disk, or a semiconductor memory card. Such a removable recording medium can be provided as so-called packaged software.

Also, the programs may be installed on a computer via a removable recording medium, and may be transferred from a download site to a computer via a network such as a LAN (Local Area Network) or the Internet in a wireless or wired manner. The computer receives the programs transferred in that manner, and can install the programs on a recording medium such as an internal hard disk.

Further, the present technique should not be limited to the above example of the technique. The above example of the technique is disclosed in the form of an example, and it is obvious that those skilled in the art can make changes and alternatives to the embodiment without departing from the scope of the technique. That is, the claims should be taken into consideration in determining the scope of the present technique.

### Industrial Applicability

In the terminal device, the external device, the information processing methods, the programs, and the information processing system according to this technique, a request for content corresponding to a date and time input from a user is issued from a terminal device to an external device, and the content selected and supplied based on the life rhythm of the user on the input date and time by the external device in response to the content request is presented. In response to a content request of the user from the terminal device, the content that matches the life rhythm of the user is selected from a content storing unit or a transmitted content storing unit by the external device, and is transmitted to the terminal device by the external device. The transmitted content storing unit stores content selected from the content storing unit in accordance with the life rhythm of the user, and the stored content is associated with dates and times. Accordingly, information that matches the life rhythm of the user at a desired time can be readily made available. For example, health care content concerning food, exercise, or the like can be readily made available in accordance with the life rhythm of the user at a desired time. Also, the frequency of use of the service providing the content can be made higher, and the service can be used regularly.

### Reference Signs List

10 Information processing system
20 Terminal device
21 Information collecting unit
22 User interface unit
23 Display unit
24, 47 Communication unit
25 Control unit
30 Life rhythm analyzing unit
31 Collected information storing unit
32 Analysis processing unit
33 Analysis results storing unit
40 Server device
41 Content registering unit
42 Content storing unit
43 Environmental information acquiring unit
44 Environmental information storing unit
45 Content transmitting unit
45 Content transmitting unit
46 Transmitted content storing unit
50 Network
200 Housing
211 Movement sensor
212 Location detecting unit
221, 222, 223 Operating switch
224 Touch panel

## Claims

1. An information processing system (10) comprising:
a user interface (22) that receives an input by a user;
a life rhythm analyzing unit (30) that analyzes the user's life rhythm based on information collected by a sensor (211) for the analysis to obtain an action pattern analysis result comprising frequency values indicating which action is often taken at what time; and
a content transmitting unit (45) that acquires the input and analysis result of the life rhythm of the user from the life rhythm analyzing unit and provides health-related advice information based on the input and the analysis result, wherein
the input comprises a time and information indicating a physical attribute of the user;
the content transmitting unit determines an action probability based on the time and the frequency value;
the content transmitting unit determines a category of health-related advice information based on the determined action probability; and
the content transmitting unit acquires the health-related advice information to be provided based on the determined category of health-related advice information, the input information indicating a physical attribute of the user and the analysis result.

2. The information processing system of claim 1, wherein
the input further corresponds to information indicating content of interest to the user.

3. The information processing system of claim 1, wherein
the input further corresponds to a selection of at least one of a plurality of categories of content.

4. The information processing system of claim 1, wherein
the determined action probability corresponds to at least one of exercising, eating and sleeping.

5. The information processing system of claim 1, wherein
the content transmitting unit determines a target level corresponding to the determined category based on the analysis result.

6. The information processing system of claim 4, wherein the content transmitting unit acquires the health-related advice information to be provided based on the determined target level.

7. The information processing system of claim 1, further comprising:
an environmental information acquiring unit that acquires environmental information corresponding to the user.

8. The information processing system of claim 1, wherein
the user interface is further configured to receive an action input by a user, and
the life rhythm analyzing unit analyzes the user's life rhythm based on the action input by the user.

9. The information processing system of claim 8, wherein
the action input by the user corresponds to information indicating at least one of a wake-up time of the user, a bedtime of the user, a diet of the user, and a category of exercise by the user.

10. The information processing system of claim 1, wherein
the health-related advice information provided by the transmitting unit is a Uniform Resource Locator (URL).

11. The information processing system of claim 1, further comprising:
a mobile terminal that includes the user interface and the life rhythm analyzing unit; and
a server that includes the content transmitting unit, wherein
the mobile terminal further includes an interface that sends the input to the server and receives the provided health-related advice information from the server.

12. The information processing system of claim 1, further comprising:
a mobile terminal that includes the user interface and a communication interface that sends the input to a server; and
the server that includes the life rhythm analyzing unit and the content transmitting unit and a communication interface that sends the provided health-related advice information to the mobile terminal.

13. An information processing method performed by an information processing system (10), the method comprising:
receiving, at a user interface (22), an input by a user;
analyzing (ST33) the user's life rhythm based on information having been collected by a sensor (211) for the analysis to obtain (ST34) an action pattern analysis result comprising frequency values indicating which action is often taken at what time;
acquiring the input and analysis result of the analyzing; and
providing health-related advice information based on the input and the analysis result, in which the input comprises a time (ST14) and information indicating a physical attribute of the user, and the step of providing health-related advice information comprises in turn the sub-steps of:
determining (ST45) an action probability based on the time and the frequency value;
determining (ST50, ST55, ST60) a category of health-related advice information based on the determined action probability; and
acquiring (ST64) the health-related advice information to be provided based on the determined category of health-related advice information, the input information indicating a physical attribute of the user and the analysis result.

14. A computer program product comprising code means adapted to perform all the steps of any preceding method claim, when said computer program product is run on a data processing system.

## Patentansprüche

1. Informationsverarbeitungssystem (10), umfassend:
eine Benutzerschnittstelle (22), die eine Eingabe durch einen Benutzer empfängt;
eine Lebensrhythmus-Analyseeinheit (30), die den Lebensrhythmus des Benutzers basierend auf Informationen, erfasst durch einen Sensor (211) für die Analyse, analysiert, um ein Aktionsmuster-Analyseergebnis zu erhalten, umfassend Häufigkeitswerte, die angeben, welche Aktion häufig zu welcher Zeit durchgeführt wird; und
eine Inhaltsübertragungseinheit (45), die die Eingabe und das Analyseergebnis des Lebensrhythmus des Benutzers von der Lebensrhythmus-Analyseeinheit erfasst und gesundheitsbezogene Ratschlaginformationen basierend auf der Eingabe und dem Analyseergebnis bereitstellt, wobei
die Eingabe eine Zeit und Informationen, die ein physikalisches Attribut des Benutzers angeben, umfasst;
die Inhaltsübertragungseinheit eine Aktionswahrscheinlichkeit basierend auf der Zeit und dem Häufigkeitswert bestimmt;
die Inhaltsübertragungseinheit eine Kategorie von gesundheitsbezogenen Ratschlaginformationen basierend auf der bestimmten Aktionswahrscheinlichkeit bestimmt; und
die Inhaltsübertragungseinheit die gesundheitsbezogenen Ratschlaginformationen, die bereitzustellen sind, basierend auf der bestimmten Kategorie von gesundheitsbezogenen Ratschlaginformationen, den Eingabeinformationen, die ein physikalisches Attribut des Benutzers angeben, und dem Analyseergebnis erfasst.

2. Informationsverarbeitungssystem nach Anspruch 1, wobei die Eingabe ferner mit Informationen, die Inhalt angeben, der für den Benutzer von Interesse ist, korrespondiert.

3. Informationsverarbeitungssystem nach Anspruch 1, wobei die Eingabe ferner mit einer Auswahl mindestens einer einer Vielzahl von Inhaltskategorien korrespondiert.

4. Informationsverarbeitungssystem nach Anspruch 1, wobei die bestimmte Aktionswahrscheinlichkeit mit mindestens einem von Trainieren, Essen und Schlafen korrespondiert.

5. Informationsverarbeitungssystem nach Anspruch 1, wobei die Inhaltsübertragungseinheit ein Zielniveau korrespondierend mit der bestimmten Kategorie basierend auf dem Analyseergebnis bestimmt.

6. Informationsverarbeitungssystem nach Anspruch 4, wobei die Inhaltsübertragungseinheit gesundheitsbezogene Ratschlaginformationen, die bereitzustellen sind, basierend auf dem bestimmten Zielniveau erfasst.

7. Informationsverarbeitungssystem nach Anspruch 1, ferner umfassend:
eine Umgebungsinformationen-Erfassungseinheit, die mit dem Benutzer korrespondierende Umgebungsinformationen erfasst.

8. Informationsverarbeitungssystem nach Anspruch 1, wobei
die Benutzerschnittstelle ferner konfiguriert ist zum Empfangen einer durch einen Benutzer eingegebenen Aktion; und
die Lebensrhythmus-Analyseeinheit den Lebensrhythmus des Benutzers basierend auf einer durch den Benutzer eingegebenen Aktion analysiert.

9. Informationsverarbeitungssystem nach Anspruch 8, wobei
die durch den Benutzer eingegebene Aktion mit Informationen korrespondiert, die mindestens eines einer Aufwachzeit des Benutzers, einer Schlafenszeit des Benutzers, einer Ernährungsweise des Benutzers und einer Kategorie von Training durch den Benutzer angeben.

10. Informationsverarbeitungssystem nach Anspruch 1, wobei
die durch die Übertragungseinheit bereitgestellten gesundheitsbezogenen Ratschlaginformationen ein Uniform Resource Locator (URL) sind.

11. Informationsverarbeitungssystem nach Anspruch 1, ferner umfassend:
ein mobiles Endgerät, das die Benutzerschnittstelle und die Lebensrhythmus-Analyseeinheit enthält; und
einen Server, der die Inhaltsübertragungseinheit enthält, wobei
das mobile Endgerät ferner eine Schnittstelle enthält, die die Eingabe an den Server sendet und die bereitgestellten gesundheitsbezogenen Ratschlaginformationen von dem Server empfängt.

12. Informationsverarbeitungssystem nach Anspruch 1, ferner umfassend:
ein mobiles Endgerät, das die Benutzerschnittstelle und eine Kommunikationsschnittstelle, die die Eingabe an einen Server sendet, enthält; und
einen Server, der die Lebensrhythmus-Analyseeinheit und die Inhaltsübertragungseinheit und eine Kommunikationsschnittstelle, die die bereitgestellten gesundheitsbezogenen Ratschlaginformationen an das mobile Endgerät sendet, enthält.

13. Informationsverarbeitungsverfahren, durchgeführt von einem Informationsverarbeitungssystem (10), das Verfahren umfassend:
Empfangen, an einer Benutzerschnittstelle (22), einer Eingabe durch einen Benutzer;
Analysieren (ST33) des Lebensrhythmus des Benutzers basierend auf Informationen, die durch einen Sensor (211) für die Analyse erfasst wurden, um ein Aktionsmuster-Analyseergebnis zu erhalten (ST34), umfassend Häufigkeitswerte, die angeben, welche Aktion häufig zu welcher Zeit durchgeführt wird;
Erfassen der Eingabe und des Analyseergebnisses der Analyse; und
Bereitstellen von gesundheitsbezogenen Ratschlaginformationen basierend auf der Eingabe und dem Analyseergebnis,
wobei die Eingabe eine Zeit (ST14) und Informationen, die ein physikalisches Attribut des Benutzers angeben, umfasst, und
der Schritt des Bereitstellens von gesundheitsbezogenen Informationen wiederum die folgenden Teilschritte umfasst:
Bestimmen (ST45) einer Aktionswahrscheinlichkeit basierend auf der Zeit und dem Häufigkeitswert;
Bestimmen (ST50, ST55, ST60) einer Kategorie von gesundheitsbezogenen Ratschlaginformationen basierend auf der bestimmten Aktionswahrscheinlichkeit; und
Erfassen (ST64) der gesundheitsbezogenen Ratschlaginformationen, die bereitzustellen sind, basierend auf der bestimmten Kategorie von gesundheitsbezogenen Ratschlaginformationen, den Eingabeinformationen, die ein physikalisches Attribut des Benutzers angeben, und dem Analyseergebnis.

14. Computerprogrammprodukt, umfassend Codemittel, angepasst zum Durchführen sämtlicher der Schritte eines vorstehenden Verfahrensanspruchs, wenn das Computerprogrammprodukt auf einem Datenverarbeitungssystem ausgeführt wird.

## Revendications

1. Système de traitement d'informations (10) comprenant :
une interface utilisateur (22) qui reçoit une entrée fournie par un utilisateur ;
une unité d'analyse de rythme de vie (30) qui analyse le rythme de vie de l'utilisateur en fonction d'informations collectées par un capteur (211) en vue de leur analyse pour obtenir un résultat d'analyse de tendance d'action comprenant des valeurs de fréquence indiquant l'action qui est souvent prise souvent et à quelle heure ; et
une unité de transmission de contenu (45) qui acquiert l'entrée et le résultat d'analyse de rythme de vie de l'utilisateur en provenance de l'unité d'analyse de rythme de vie et fournit une information de conseil de santé en fonction de l'entrée et du résultat d'analyse, dans lequel
l'entrée comprend une heure et des informations indiquant un attribut physique de l'utilisateur ;
l'unité de transmission de contenu détermine une probabilité d'action en fonction de l'heure et de la valeur de fréquence ;
l'unité de transmission de contenu détermine une catégorie d'information de conseil de santé en fonction de la probabilité d'action déterminée ; et
l'unité de transmission de contenu acquiert l'information de conseil de santé à fournir en fonction de la catégorie déterminée d'information de conseil de santé, les informations d'entrée indiquant un attribut physique de l'utilisateur et le résultat d'analyse.

2. Système de traitement d'informations selon la revendication 1, dans lequel
l'entrée correspond en outre à des informations indiquant un contenu d'intérêt pour l'utilisateur.

3. Système de traitement d'informations selon la revendication 1, dans lequel
l'entrée correspond en outre à une sélection d'au moins une d'une pluralité de catégories de contenu.

4. Système de traitement d'informations selon la revendication 1, dans lequel
la probabilité d'action déterminée correspond à au moins une d'une action d'exercice physique, de nutrition et de sommeil.

5. Système de traitement d'informations selon la revendication 1, dans lequel
l'unité de transmission de contenu détermine un niveau cible correspondant à la catégorie déterminée en fonction du résultat d'analyse.

6. Système de traitement d'informations selon la revendication 4, dans lequel
l'unité de transmission de contenu acquiert l'information de conseil de santé à fournir en fonction du niveau cible déterminé.

7. Système de traitement d'informations selon la revendication 1, comprenant en outre :
une unité d'acquisition d'informations environnementales qui acquiert des informations environnementales correspondant à l'utilisateur.

8. Système de traitement d'informations selon la revendication 1, dans lequel
l'interface utilisateur est configurée en outre pour recevoir une entrée d'action par un utilisateur, et
l'unité d'analyse de rythme de vie analyse le rythme de vie de l'utilisateur en fonction de l'entrée d'action par l'utilisateur.

9. Système de traitement d'informations selon la revendication 8, dans lequel
l'entrée d'action par l'utilisateur correspond à des informations indiquant au moins d'une heure de réveil de l'utilisateur, d'une heure de coucher de l'utilisateur, d'un régime de l'utilisateur, et d'une catégorie d'exercice par l'utilisateur.

10. Système de traitement d'informations selon la revendication 1, dans lequel
l'information de conseil de santé fournie par l'unité de transmission est une adresse URL (Uniform Resource Locator).

11. Système de traitement d'informations selon la revendication 1, comprenant en outre :
un terminal mobile qui comporte l'interface utilisateur et l'unité d'analyse de rythme de vie ; et
un serveur qui comporte l'unité de transmission de contenu, dans lequel
le terminal mobile comporte en outre une interface qui envoie l'entrée au serveur et reçoit l'information de conseil de santé fournie en provenance du serveur.

12. Système de traitement d'informations selon la revendication 1, comprenant en outre :
un terminal mobile qui comporte l'interface utilisateur et une interface de communication qui envoie l'entrée à un serveur ; et
le serveur qui comporte l'unité d'analyse de rythme de vie et l'unité de transmission de contenu et une interface de communication qui envoie l'information de conseil de santé fournie au terminal mobile.

13. Procédé de traitement d'informations exécuté par un système de traitement d'informations (10), le procédé comprenant :
la réception, au niveau d'une interface utilisateur (22), d'une entrée fournie par un utilisateur ;
l'analyse (ST33) du rythme de vie de l'utilisateur en fonction d'informations ayant été collectées par un capteur (211) en vue de leur analyse pour obtenir (ST34) un résultat d'analyse de tendance d'action comprenant des valeurs de fréquence indiquant l'action qui est souvent prise et à quelle heure ; et
l'acquisition de l'entrée et du résultat d'analyse de l'analyse ; et
la fourniture d'une information de conseil de santé en fonction de l'entrée et du résultat d'analyse,
dans lequel l'entrée comprend une heure (ST14) et des informations indiquant un attribut physique de l'utilisateur, et
l'étape de fourniture d'une information de conseil de santé comprend à la fois les sous-étapes suivantes :
la détermination (ST45) d'une probabilité d'action en fonction de l'heure et de la valeur de fréquence ;
la détermination (ST50, ST55, ST60) d'une catégorie d'information de conseil de santé en fonction de la probabilité d'action déterminée ; et
l'acquisition (ST64) de l'information de conseil de santé à fournir en fonction de la catégorie déterminée d'information de conseil de santé, les informations d'entrée indiquant un attribut physique de l'utilisateur et le résultat d'analyse.

14. Produit-programme informatique comprenant un moyen de code adapté pour exécuter toutes les étapes de n'importe quelle revendication de procédé précédente, quand ledit produit-programme informatique est exécuté sur un système de traitement de données.
